(19) Europäisches Patentamt
European Patent Office
Office européen des brevets

(11) Publication number: **0 313 033 A2**

(12) **EUROPEAN PATENT APPLICATION**

(21) Application number: **88117425.4**

(51) Int. Cl.[4]: **C07K 5/08 , A61K 37/64**

(22) Date of filing: **19.10.88**

(30) Priority: **21.10.87 YU 1936/87**

(43) Date of publication of application:
**26.04.89 Bulletin 89/17**

(84) Designated Contracting States:
**AT BE CH DE ES FR GB IT LI NL SE**

(71) Applicant: **KRKA, tovarna zdravil, n.sol.o**
**Cesta herojev 45**
**YU-68000 Novo Mesto(YU)**

(72) Inventor: **Merslavic, Marjo, Dr.**
**Cesta herojev 30**
**YU-68000 Novo mesto(YU)**
Inventor: **Zupet, Pavel, Dr.**
**Mackovec 12A**
**YU-68000 Novo mesto(YU)**
Inventor: **Florjanic, Miha**
**Labanova 42**
**YU-68000 Novo mesto(YU)**
Inventor: **Zalar, Stanislav**
**Skrjance 3**
**YU-68000 Novo mesto(YU)**

(74) Representative: **Deufel, Paul, Dr.**
**Patentanwälte Müller-Boré, Deufel, Schön, Hertel, Lewald, Otto Isartorplatz 6 Postfach 26 02 47**
**D-8000 München 26(DE)**

(54) **L-Aspartyl-L-Phenylalanine derivative and a process for preparing the same.**

(57) There is disclosed a novel L-aspartyl-L-phenylalanine derivative of the formula I

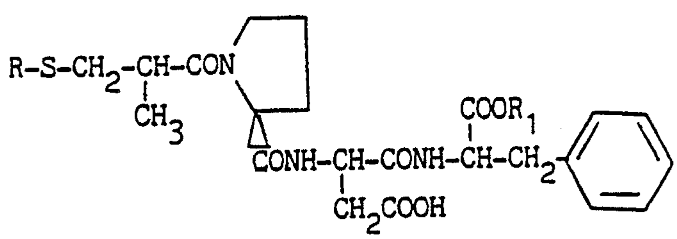

I

wherein R is hydrogen or $CH_3CO$ and $R_1$ is hydrogen or methyl, and pharmaceutically acceptable salts thereof. It is prepared in such a way that a compound of the formula II

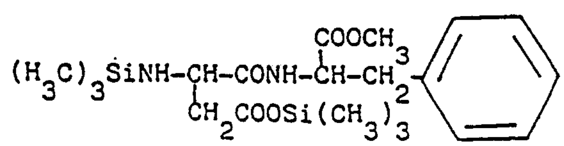

II

EP 0 313 033 A2

is condensed with an activated form of an acid of the formula III

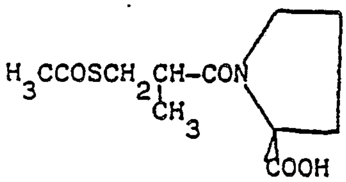

III

in a water-immiscible solvent such as methylene chloride in the temperature range from -20° to 0° C, optionally the obtained compound of the formula I, wherein R represents a $CH_3CO$ group and $R_1$ represents a methyl group, is converted into the compound of the formula I, wherein R and $R_1$ represent a hydrogen atom, and then it is optionally converted into pharmaceutically acceptable salts thereof.

The compound of the formula I inhibits the conversion of angiotensine 1 into angiotensine 2, which plays an important role in regulating blood pressure.

# L-ASPARTYL-L-PHENYLALANINE DERIVATIVE AND A PROCESS FOR PREPARING THE SAME

## Technical Field

The present invention belongs to the field of organic chemistry and relates to an L-aspartyl-L-phenylalanine derivative of the formula I

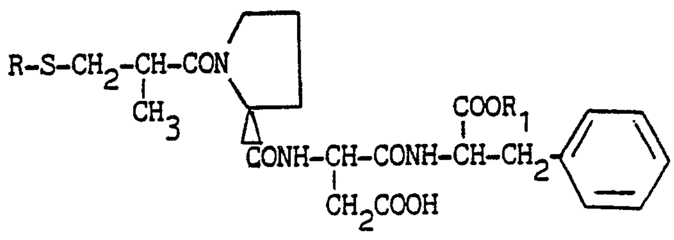

I

wherein R represents a hydrogen atom or a $CH_3CO$ group and $R_1$ represents a hydrogen atom ot a methyl group,
and to pharmaceutically acceptable salts thereof, such as sodium, potassium, calcium, dicyclohexyl-amine, procaine, lysine and arginine salts, as well as to a process for preparing the same.

The derivative of formula I inhibits the conversion of decapeptide angiotensine 1 into angiotensine 2, which plays a role in regulating blood pressure. The enzyme rennin secreted by the kidneys acts upon one of the plasma proteins, namely rennin substrate - angiotensinogen. The latter is secreted by the liver and it cleaves off the decapeptide angiotensine 1. Angiotensine 1 is converted into angiotensine 2 by means of the enzyme angiotensinase. The derivatives of the compound of formula I are inhibitors of the enzyme angiotensinase.

## Technical Problem

There was a need to find a novel L-aspartyl-L-phenylalanine derivative having similar characteristics as captopril US 4,154,840) and its analogue alacepril (Drugs of Future, Vol 11, No. 5, 1986) as well as enalapril maleate (US 4,374,829).

## Prior Art

The derivative of the invention is a novel compound and, consequently, no processes for the preparation thereof exist.

## Description of the Inventive Solution

The object of the invention is a novel L-aspartyl-L-phenylalanine derivative of the formula I and pharmaceutically acceptable salts thereof.

According to the invention it is prepared in such a way that a compound of the formula II

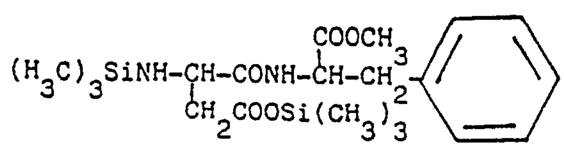

II

is condensed with an activated form of an acid of the formula III

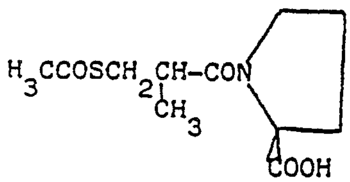

III

in a water-immiscible solvent such as methylene chloride in the temperature range from -20° to 0° C, optionally the obtained compound of the formula I, wherein R represents a $CH_3CO$ group and $R_1$ represents a methyl group, is converted into the compound of the formula I, wherein R and $R_1$ represent a hydrogen atom, and then it is optionally converted into pharmaceutically acceptable salts thereof. The radicals R and $R_1$ are converted into hydrogen atoms in a conventional manner.

The compound of the formula II is commercially available (FLUKA product), whereas the compound of the formula III may be prepared according to the process disclosed in Yugoslavian Patent Application P 1912/85.

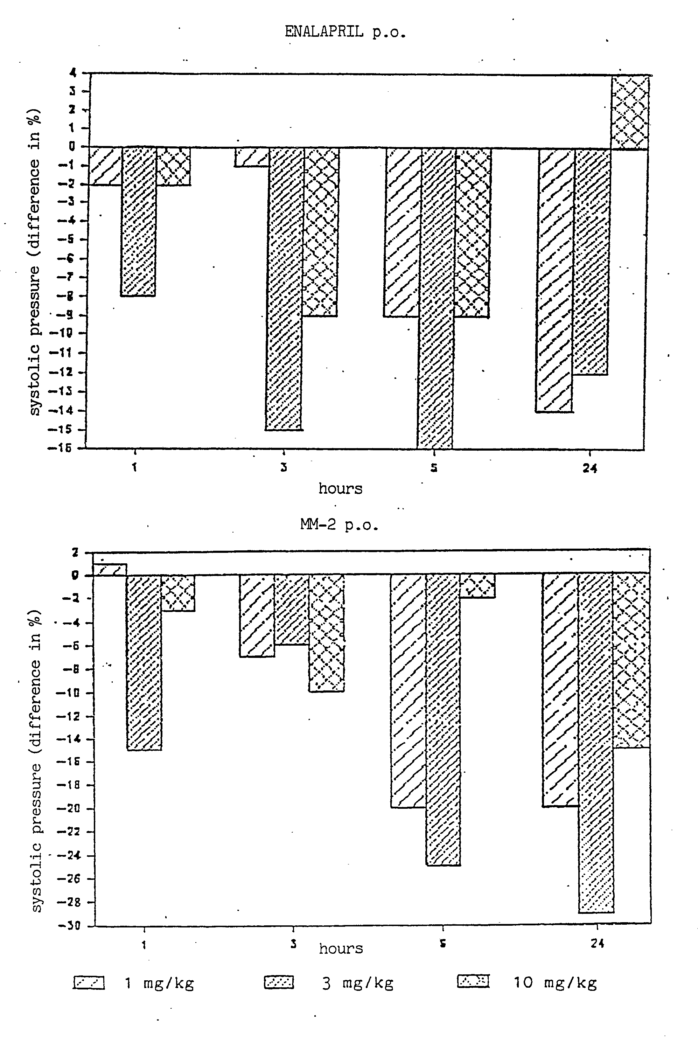

The novel compound of the invention belongs to the inhibitor group ACE - angiotensine converting enzyme - (dipeptidyl carboxy peptidase) and acts upon the cardiovascular system reducing arterial pressure. The hypotensive action was measured in DOCA/NaCl hypertensive rats according to Deitchman (1980). The reduction of systolic pressure was compared to that of enalapril maleate and the results are given in the enclosed drawing.

It is evident from the results that both ACE inhibitors induce a siginificant reduction of systolic pressure in DOCA/NaCl hypertensive rats. It takes from 1 to 3 hours for the action to begin. The maximum reduction of systolic pressure is more pronounced under the influence of the substance MM-2 (sodium salt of the compound of the formula I) up to 29 %) than under the influence of enalapril maleate (up to 16 %).

Hence the novel compound of the formula I is a potential active substance in drugs for the therapy of cardiovascular diseases, especially of hypertension.

The invention is disclosed in detail by means of the following Examples, it is, however, not limited thereto.

Example 1

//1-(3-acetylthio-2-D-methylpropanoyl)/-L-prolyl/-alpha-L-aspartyl-L-phenylalanine methyl ester

To a suspension of alpha-L-aspartyl-L-phenylalanine methyl ester (1.47 g) in methylene chloride (40 ml) there were added triethylamine (1.4 ml) and trimethylchlorosilane (1.3 ml) and it was heated at the boiling point of the suspension for 3 hours. The cooled suspension was added at 0° C to a mixed anhydride of 1-(3-acetylthio-2-D-methylpropanoyl)-L-proline, which was previously prepared in such a way that 1-(3-acetylthio-2-D-methylpropanoyl)-L-proline (1.1 g) was dissolved in methylene chloride (10 ml), cooled to 0° C, N-methylmorpholine (0.6 ml) and ethyl chloroformiate (0.5 ml) were added thereto and it was left to stir at the same temperature for 1 hour. The condensation reaction ran for 2 hours at 0° C, then water (30 ml) was added, it was alkalinized with potassium hydrogen carbonate to pH 8 and the layers were separated. The aqueous layer was acidified with 2 N hydrochloric acid to pH 2 and extracted with ethyl acetate (3 x 30 ml). The combined ethyl acetate layers were dried with sodium sulfate and evaporated. There was obtained the title product (2 g; 81 %), m.p. 132-135° C.

| Elemental analysis for $C_{25}H_{33}N_3O_8S$ | | | | | | |
|---|---|---|---|---|---|---|
| calc.: | C | 56.06 | H | 6.21 | N | 7.85 |
| found: | C | 56.53 | H | 6.24 | N | 7.92 |

Example 2

//1-(3-acetylthio-2-D-methylpropanoyl)/-L-prolyl/-alpha-L-aspartyl-L-phenylalanine methyl ester sodium salt

//1-(3-acetylthio-2-D-methylpropanoyl)/-L-prolyl/-alpha-L-aspartyl-L-phenyalanine methyl ester (4.2 g) was suspended in ethanol (100 ml) and it was heated to 50 °C up to a total dissolution. Then sodium 2-ethyl-hexanoate (1.4 g) was added and ethanol was evaporated. Ether was added to the residue and the precipitate was filtered off. There was obtained the title product (4.0 g; 90 %), m.p. 150-155 °C.

| Elemental analysis for $C_{25}H_{32}N_3O_8SNa.1/2\ H_2O$ | | | | | | |
|---|---|---|---|---|---|---|
| calc.: | C | 53.00 | H | 5.87 | N | 7.42 |
| found: | C | 53.10 | H | 5.87 | N | 7.43 |

## Claims

1. L-aspartyl-L-phenylalanine derivative of the formula I

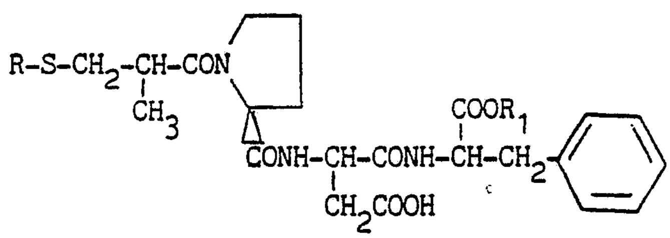

I

wherein R represents a hydrogen atom or a $CH_3CO$ group and $R_1$ represents a hydrogen atom or a methyl group, and pharmaceutically acceptable salts thereof.

2. Process for preparing L-aspartyl-L-phenylalanine derivative of the formula I

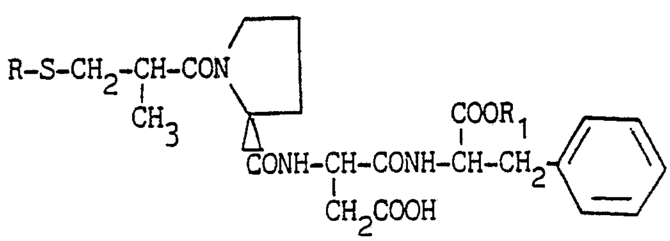

I

wherein R represents a hydrogen atom or a $CH_3CO$ group and $R_1$ represents a hydrogen atom or a methyl group, and pharmaceutically acceptable salts thereof, characterized in that a compound of the formula II

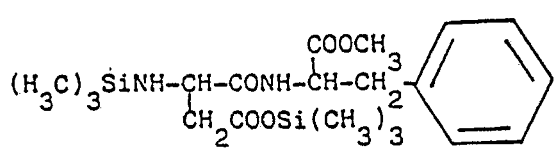

II

is condensed with an activated form of an acid of the formula III

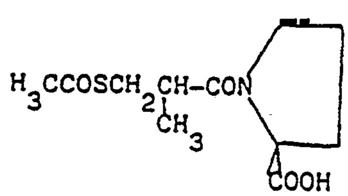

III

in a water-immiscible solvent such as methylene chloride in the temperature range from -20° to 0°C, optionally the obtained compound of the formula I, wherein R represents a $CH_3CO$ group and $R_1$ represents a methyl group, is converted into the compound of the formula I, wherein R and $R_1$ represent a hydrogen atom, and then it is optionally converted into pharmaceutically acceptable salts thereof.

3. A medicine which contains a novel derivative of L-aspartyl-L-phenylalanine derivative according to claim 1 or a pharmaceutically acceptable salt thereof as the active substance.

ENALAPRIL p.o.

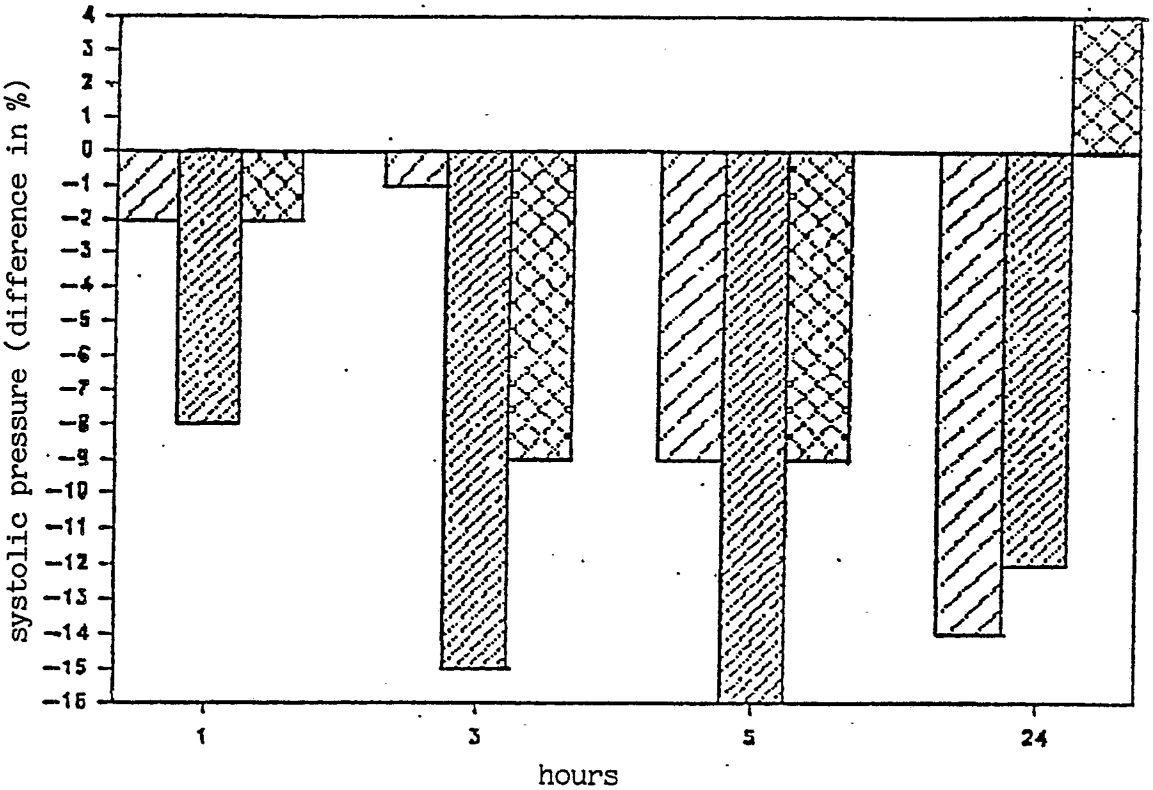


MM-2 p.o.